# EUROPÄISCHE PATENTANMELDUNG

(11) **EP 0 823 424 A1**
(43) Veröffentlichungstag der Anmeldung: **11.02.1998**
(21) Anmeldenummer: 97113283.2
(22) Anmeldetag: 01.08.1997
(51) Int. Cl.: C07D 215/48, C07D 401/12

(54) **Verfahren zur Herstellung von Chinargin**

(30) Priorität: 09.08.1996 EP 96112866
(71) Anmelder: F. HOFFMANN-LA ROCHE AG, 4070 Basel (CH)
(72) Erfinder: Göhring, Wolfgang, 79585 Steinen (DE)
(74) Vertreter: Witte, Hubert, Dr.

(57) **Zusammenfassung**

Die vorliegende Anmeldung betrifft ein Verfahren zur Herstellung von Chinarginderivaten der Formel I 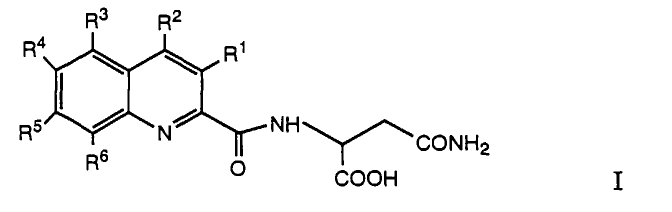 wobei
R¹, R², R³, R⁴, R⁵ und R⁶ unabhängig voneinander Wasserstoff, Halogen, C₁₋₃-Alkyl, Halogen-C₁₋₃-Alkyl, Hydroxy-C₁₋₃-Alkyl, C₁₋₃-Alkoxy, C₁₋₃-Alkylthio, C₁₋₃-Alkylthio-C₁₋₃-Alkyl, Nitro oder Trifluoromethyl, bevorzugt jedoch Wasserstoff, sind.

## Beschreibung

Die vorliegende Erfindung betrifft ein neues Verfahren zur Herstellung von Chinargin (N-(2-Chinolylcarbonyl)-asparagin) und Chinarginderivaten.

Chinargin ist an sich bekannt und beispielsweise in der Europäischen Patentanmeldung Nr. 611,774 beschrieben. Es ist ein wertvolles Zwischenprodukt zur Herstellung pharmakologisch aktiver Verbindungen. So kann Chinargin wie in Beispiel 7 der genannten Europäischen Patentanmeldung dargestellt in pharmakologisch aktive Verbindungen überführt werden, die sich vor allem zur Behandlung viraler Infektionen eignen, insbesondere solcher Infektionen, die durch HIV oder andere Retroviren verursacht werden.

Zur Herstellung von Chinaldinsäurederivaten des obengenannten Typs sind verschiedene Reaktionswege beschrieben worden. Davis zum Beispiel beschreibt die normalerweise in guten Ausbeuten verlaufende Umsetzung von Chinaldinsäurechlorid mit primären und sekundären Aminen, Aminosäuren und Aminosäureestern unter Schotten-Baumann Bedingungen (J. Am. Chem. Soc. (1959) , 24, 1691-1694). Bei der Schotten-Baumann Reaktion wird die zu acylierende Substanz in 10%iger Natronlauge mit einem Säurechlorid geschüttelt, bis dieses verbraucht ist. Man arbeitet mit einem grossen Ueberschuss an Lauge und Säurechlorid. Dieses Verfahren führt jedoch bei der Herstellung von Chinargin und seinen Derivaten nur zu mässigen Ausbeuten.

Anderson et al. beschreiben die Verwendung von N-Hydroxysuccinimidestern bei Peptidsynthesen (Anderson et al. (1964) J. Am. Chem. Soc. 86, 1839 - 1842). Aufgrund der grösseren Wasserlöslichkeit sind diese Ester im allgemeinen besser für die Synthese geeignet als die entsprechenden N-Hydroxyphthalimidester. Die Herstellung der N-Acyl-Aminosäure-N-Hydroxysuccinimidester erfolgt im allgemeinen durch Umsetzen einer N-Acyl-Aminosäure mit N-Hydroxysuccinimid nach der gemischten Anhydridmethode, vornehmlich aber mittels Dicyclohexylcarbodiimid (P. Stelzel in Houben-Weyl: "Methoden der organischen Chemie; Synthese von Peptiden", Band 15, Teil 2, S. 214, (1974)). Die Hydroxysuccinimidester werden dann in einem organisch-wässrigen Lösungsmittelgemisch mit der entsprechenden Aminosäure umgesetzt.

Wendlberger beschreibt ebenfalls die Anwendung von Hydroxysuccinimidestern bei der Peptidsynthese (Houben-Weyl: "Methoden der organischen Chemie; Synthese von Peptiden", Band. 15, Teil 2 S. 130 (1974)). Die hohe Aminolysierbarkeit der N-Hydroxysuccinimidester gegenüber der geringen Hydrolyse- und Alkoholyse-Empfindlichkeit gestattet es, Peptidsynthesen nicht nur in organischen Lösungsmitteln, sondern auch in organisch-wässrigen Lösungsmittelgemischen, wie Ethanol/Wasser, Dioxan/Wasser usw. durchzuführen. Peptidsynthesen mit N-Hydroxysuccinimidestern können auch im Zweiphasensystem wie Dichlormethan/Wasser, etc. durchgeführt werden.

In der Internationalen Patentanmeldung WO95/20962 wird die Herstellung von 2S-N(Chinolin-2-ylcarbonyl)amino-3-oxo-3-aminopropansäure beschrieben. Hier erfolgt die Umsetzung des entsprechenden Pentafluorphenylesters mit Asparagin in einem Dioxan-Wasser-Gemisch.

Allerdings weisen alle genannten Verfahren bei der Herstellung von Chinargin und den entsprechenden Derivaten erhebliche Nachteile auf. So verläuft die Reaktion bei weitem nicht quantitativ. Dies ist in erster Linie auf eine Nebenproduktbildung durch Hydrolyse zurückzuführen. Folglich muss auch die Reaktionsmischung einer aufwendigen Aufarbeitung unterzogen werden. Ausserdem entstehen bei der Herstellung von Chinargin bzw. Chinarginderivaten problematische Nebenprodukte wie das toxische Pentafluorphenol (WO95/20962), so dass die grosstechnische Herstellung mittels der Methoden des Standes der Technik nicht zufriedenstellend zu bewerkstelligen ist.

Das erfindungsgemässe Verfahren vermeidet die aus dem Stand der Technik bekannten Nachteile.

Das vorliegende Verfahren zur Herstellung von Chinargin und Chinarginderivaten der Formel I, 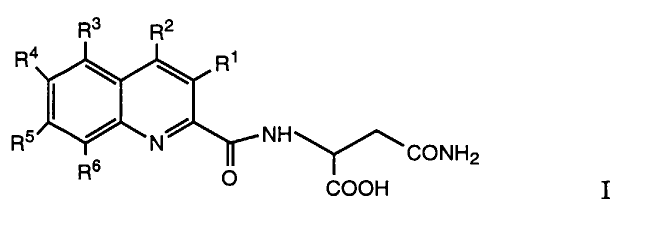 wobei
R¹, R², R³, R⁴, R⁵ und R⁶ unabhängig voneinander Wasserstoff, Halogen, C₁₋₃-Alkyl, Halogen-C₁₋₃-Alkyl, Hydroxy-C₁₋₃-Alkyl, C₁₋₃-Alkoxy, C₁₋₃-Alkylthio, C₁₋₃-Alkylthio-C₁₋₃-Alkyl, Nitro oder Trifluoromethyl, bevorzugt jedoch Wasserstoff, sind,
beruht auf der Umsetzung von Succinimidesterderivaten der Formel II, 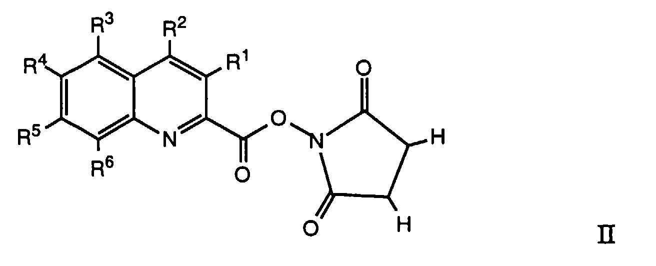
wobei R¹, R², R³, R⁴, R⁵ und R⁶ die obengenannte Bedeutung besitzen,
mit Asparagin bzw. Asparaginsalzen in einem wässrigen Reaktionsmedium bei einem neutralem pH-Wert.

Das Verfahren eignet sich insbesondere zur Herstellung von Chinargin (N-(2-Chinolylcarbonyl)-asparagin). In Abhängigkeit vom eingesetzten Asparagin bzw. Asparaginsalz erhält man das S- bzw. R-Chinargin.

Die entsprechenden Ausgangsstoffe der Formel II sind bekannt können beispielsweise nach der von Anderson et al. (loc. cit.) beschriebenen Methode hergestellt werden. Die Herstellung des Succinimidesters erfolgt zum Beispiel durch Umsetzung der entsprechenden Säurekomponente mit N-Hydroxysuccinimid unter Zusatz von Dicyclohexylcarbodiimid.

Das erfindungsgemässe Verfahren ist dadurch gekennzeichnet, dass man den Succinimidester der Formel II in wässrigem Medium bei einem ungefähr neutralem pH-Wert umsetzt. Vorteilhafterweise sollte die Umsetzung bei einem pH-Wert zwischen 6 und 8, bevorzugt bei einem pH-Wert von ca. 7 stattfinden. Der pH-Wert kann durch gängige Puffersysteme eingestellt werden, zum Beispiel Carbonat- oder Phosphatpuffer. Bevorzugt ist eine wässerige Natriumhydrogencarbonatlösung.

Die Umsetzung des N-Succinimidesters der Formel II erfolgt bei ca. 20 bis 70°C, bevorzugt bei 50°C. Die Reaktionsdauer beträgt 2 bis 8 Stunden, wobei im Schnitt eine Reaktionsdauer von 2,5 Stunden für den quantitativen Umsatz ausreichend ist.

Zur Aufarbeitung des Reaktionsansatzes wird bei erhöhter Temperatur Methanol und konzentrierte Salzsäure zugesetzt. Die konzentrierte Salzsäure wird so zudosiert, dass sich ein pH-Wert von ca. 2,5 bis 3 einstellt. Vorteilhafterweise erfolgt die Zugabe einer ca. äquimolaren Menge HCl bei 30 bis 70°C, bevorzugt bei 50°C. Anschliessend lässt man das Reaktionsgemisch für etwa 2 bis 8 Stunden, bevorzugt 2,5 Stunden mit konstanter Abkühlgeschwindigkeit auf 0 bis 30°C, bevorzugt auf ca. 10°C, abkühlen. Anschliessend kann das Reaktionsprodukt abfiltriert werden.

Nach dieser Aufarbeitung fällt das Reaktionsprodukt der Formel I mit einer Ausbeute von 90 bis 93 % und damit nahezu quantitativ an. Für Folgereaktionen, etwa zur Herstellung von HIV-Inhibitoren, ist keine weitere Aufreinigung erforderlich.

Weiterhin umfasst die vorliegende Erfindung die Herstellung pharmazeutisch aktiver Substanzen. Beispielsweise kann N-(2-Chinolylcarbonyl)-L-asparagin durch Umsetzung mit 2-[3(S)-Amino-2(R)-hydroxy-4-phenylbutyl]-N-tert.butyl-decahydro-(4aS,8aS)-isochinolin- 3(S)carboxamid, das aus der Europäischen Patentanmeldung Nr. 635,493 bekannt ist, in Gegenwart eines Kupplungsreagenzes wie z. B. eines Carbodiimids und einer N-Hydroxyverbindung, wobei die N-Hydroxyverbindung in katalytischer Menge vorliegt, umgesetzt werden. Wie in Beispiel 7 der Europäischen Patentanmeldung Nr. 611,774 beschrieben, können in Gegenwart von Dicyclohexylcarbodiimid mittels einer katalytischen Menge 1-Hydroxy-2(1H)-pyridon in einem inertem Lösungsmittel bzw. Lösungsmittelgemisch wie Ethylacetat/Tetrahydrofuran die obengenannten Substanzen zu N-t-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid oder zu davon abgeleiteten, pharmazeutisch geeigneten Salzen oder zu entsprechenden Estern umgesetzt werden. N-t-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid und seine genannten Derivate können, wie in den Europäischen Patentanmeldungen Nr. 346,847 und Nr. 432,695 beschrieben, als antivirale Mittel, insbesondere als HIV-Inhibitoren eingesetzt werden.

Damit umfasst die Erfindung auch ein Verfahren zur Herstellung dieser Verbindungen. Ein solches Verfahren umfasst in einem ersten Schritt die Herstellung von Chinargin wie oben beschrieben. Diese Substanz wird dann in einem Folgeschritt mit 2-[3(S)-Amino-2(R)-hydroxy-4-phenylbutyl]-N-tert.butyl-decahydro-(4aS,8aS)-isochinolin-3(S)carboxamid zu N-t-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid wie oben beschrieben oder gegebenenfalls zu einem entsprechenden Salz, wobei das entsprechende Methansulfonsäuresalz bevorzugt ist, oder zu einem Ester umgesetzt.

Die Erfindung umfasst ausserdem die Verwendung eines oben beschriebenen Verfahrens zur Herstellung von Folgeprodukten, insbesondere HIV-Proteaseinhibitoren wie zum Beispiel N-t-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid.

Weiterhin umfasst die Erfindung Substanzen, die nach einem der oben genannten Verfahren erhältlich sind.

### BEISPIELE

### Beispiel 1: Herstellung von S-Chinargin

200 g Chinaldinsäuresuccinimidester werden mit 117 g L-Asparagin-Monohydrat unter schwachem Stickstoffstrom und unter Rühren (600 U/Min) für zwei Stunden in einer wässrigen Natriumhydrogencarbonatlösung (66 g in 1,5 Liter) umgesetzt. Innerhalb von 2 Stunden wird die Innentemperatur der weissen Suspension, mit konstanter Aufheizrate, von 20°C auf 50°C erhöht. Die weisse Suspension färbt sich dabei schwach rosa. Zur Reaktionskontrolle kann eine Probe mittels HPLC auf Abwesenheit (< 0,5 %) des Chinaldinsäuresuccinimidesters geprüft werden. Bei 50°C lässt man noch 0,5 Stunden rühren. Die Reaktion ist dann beendet und dem Reaktionsgemisch werden 750 ml Methanol zugefügt. Nach 5 - 10 Minuten geht das Reaktionsgemisch fast vollständig in Lösung. Dann werden ca. 90 ml konz. Salzsäure so zudosiert, dass sich der pH des Reaktionsgemisches auf 2,5 - 3 einstellt. Das S-Chinargin fällt langsam weiss aus. Die Rührgeschwindigkeit wird beibehalten. Die Innentemperatur steigt von 50°C auf 54°C an. Ist die Salzsäure vollständig zugegeben, wird die Suspension von 54°C innerhalb von 2,5 Stunden mit konstanter Abkühlgeschwindigkeit auf 10°C gebracht und lässt bei dieser Temperatur noch 0,5 Stunden nachrühren. Die Suspension wird über eine Glassinternutsche (10 cm Durchmesser, Porösität 3) abfiltriert. Die ziemlich groben Eristalle erlauben eine schnelle Filtration. Der Reaktor wird mehrmals mit der 10°C kalten Mutterlauge ausgespült. Der Filterkuchen wird anschliessend dreimal mit jeweils insgesamt 300 ml demineralisiertem Wasser gewaschen. Der Filterkuchen wird trockengesaugt und im Vakuumschrank (15 - 20 mbar) bei 60°C für 24 Stunden getrocknet.
Ausbeute: 185,6 - 190,3 g (90 - 93 %); Reinheit: ≥ 97%.

### Beispiel 2: Herstellung von R-Chinargin

Es wird analog zu Beispiel 1 verfahren. R-Chinargin fällt mit einem Gehalt von > 99 % mit einem Drehwert von -50,1°C (1% DMF) und einem Anteil S-Chinargin von 0,1% an.

## Patentansprüche

1. Verfahren zur Herstellung von Chinargin und Chinarginderivaten der Formel I 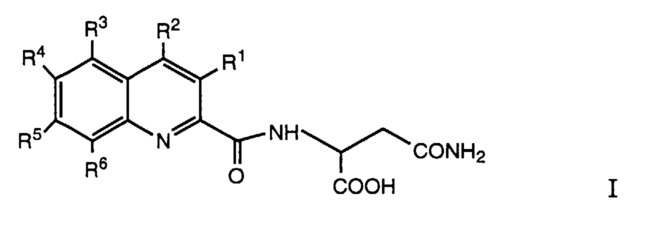 wobei
R¹, R², R³, R⁴, R⁵ und R⁶ unabhängig voneinander Wasserstoff, Halogen, C₁₋₃-Alkyl, Halogen-C₁₋₃-Alkyl, Hydroxy-C₁₋₃-Alkyl, C₁₋₃-Alkoxy, C₁₋₃-Alkylthio, C₁₋₃-Alkylthio-C₁₋₃-Alkyl, Nitro oder Trifluoromethyl sind,
dadurch gekennzeichnet, dass Succinimidesterderivate der Formel II 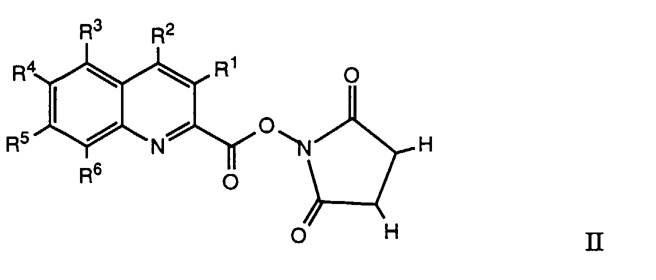
wobei R¹, R², R³, R⁴, R⁵ und R⁶ die obengenannte Bedeutung besitzen,
in einem wässrigen Reaktionsmedium bei einem neutralen pH-Wert mit Asparagin oder Asparaginsalzen umgesetzt werden.

2. Verfahren gemäss Anspruch 1, dadurch gekennzeichnet, dass R¹, R², R³, R⁴, R⁵ und R⁶ Wasserstoff sind.

3. Verfahren gemäss Anspruch 1 oder 2, dadurch gekennzeichnet, dass die Reaktion bei einem pH-Wert zwischen pH 6 und pH 8 stattfindet.

4. Verfahren gemäss Anspruch 3, dadurch gekennzeichnet, dass die Reaktion mit Natriumhydrogencarbonat gepuffert wird.

5. Verfahren gemäss Anspruch 1 oder 2, dadurch gekennzeichnet, dass die Reaktion bei einer Temperatur zwischen 20 und 70°C stattfindet.

6. Verfahren gemäss Anspruch 1 oder 2, dadurch gekennzeichnet, dass die Reaktionszeit 2 bis 8 Stunden beträgt.

7. Verfahren gemäss einem der Ansprüche 1 - 6, dadurch gekennzeichnet, dass zur Abtrennung des Reaktionsproduktes eine äquimolare Menge HCl in Methanol zugegeben wird.

8. Verfahren gemäss Anspruch 7, dadurch gekennzeichnet, dass die HCl/Methanol-Zugabe bei einer Temperatur zwischen 30 und 70 °C erfolgt.

9. Verfahren zur Herstellung von N-tert-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid oder davon abgeleiteter Salze oder Ester, durch
a) Herstellung von N-(2-Chinolylcarbonyl)-L-asparagin gemäss einem der Ansprüche 1 - 8,
b) Umsetzung des erhaltenen N-(2-Chinolylcarbonyl)-L-asparagin mit 2-[3(S)-Amino-2(R)-hydroxy-4-phenylbutyl]-N-tert.butyl-decahydro-(4aS,8aS)-isochinolin-3(S)carboxamid und
c) gegebenenfalls Ueberführung der in Schritt b) erhaltenen Substanz in ein pharmazeutisch geeignetes Salz oder in einen pharmazeutisch geeigneten Ester.

10. Verwendung eines Verfahrens gemäss einem der Ansprüche 1 bis 9 zur Herstellung von N-t-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid.
